# FASCICULE DE BREVET EUROPEEN

(11) **EP 1 584 288 B1**
(45) Date de publication et mention de la délivrance du brevet: **18.07.2007**
(21) Numéro de dépôt: 05290748.2
(22) Date de dépôt: 05.04.2005
(51) Int. Cl.: A61B 5/0205, A61N 1/365, A61B 5/08

(54) **Dispositif médical implantable actif pourvu de moyens de diagnostic de troubles respiratoires, à détection perfectionnée des cycles respiratoires artéfactés**
Aktives implantierbares medizinisches Gerät mit respiratorischen Diagnosemitteln und verbesserter Detektion von respiratorischen Artefakten
Active implantable medical device comprising means for diagnosis of respiratory conditions, having enhanced detection of respiratory artifacts

(30) Priorité: 05.04.2004 FR 0403528
(43) Date de publication de la demande: 12.10.2005
(73) Titulaire: ELA MEDICAL, 92541 Montrouge (FR)
(72) Inventeur: Poezevara, Yann, 91080 Courcouronnes (FR)
(74) Mandataire: Dupuis-Latour, Dominique

(56) Documents cités:
- EP-A- 0 702 977
- WO-A-02/087433
- US-A- 4 567 892
- US-A1- 2002 193 697
- US-B1- 6 574 507

## Description

La présente invention concerne le diagnostic des troubles respiratoires du sommeil tels que les apnées ou les hypopnées par un "dispositif médical actif implantable" tel que défini par la directive 90/385/CEE du 20 juin 1990 du Conseil des communautés européennes, notamment un dispositif tel que stimulateur cardiaque, défibrillateur/cardioverteur ou dispositif multisite.

On connaît diverses techniques pour détecter les troubles respiratoires du sommeil, techniques qui mettent en oeuvre divers types de capteurs.

Par exemple le EP-A-0 970 713/US-A-6 574 507 (ELA Medicat) diagnostique la survenue d'une apnée à partir du signal de ventilation-minute (signal VE, dit également signal MV), qui est un paramètre à prépondérance physiologique généralement obtenu par une mesure d'impédance transthoracique donnant une indication continue du rythme respiratoire du patient.

Le US 2002/0193697 décrit un dispositif ayant un objet comparable.

La détection des apnées ou des hypopnées permet de diagnostiquer des affections telles que le syndrome d'apnée du sommeil (SAS), apnée obstructive ou bien centrale, qui est une pathologie susceptible d'entraîner un certain nombre de troubles tels qu'hypersomnolence diurne, trouble du rythme cardiaque, hypertension.

Un SAS peut être défini par une récurrence importante d'apnées ou d'hypopnées associées à des signes cliniques. Une "apnée" ou "pause respiratoire" est un arrêt temporaire de la fonction respiratoire de durée supérieure à 10 secondes et survenant pendant une phase de sommeil du patient ; une "hypopnée" est définie comme une décroissance importante de la ventilation-minute, par exemple plus de 50%, par rapport à une moyenne de référence antérieure, mais sans arrêt de la fonction respiratoire (la ventilation-minute est le produit de l'amplitude par la fréquence des cycles respiratoires successifs).

La mesure de la ventilation-minute nécessaire à cette détection des apnées ou des hypopnées est réalisée par injection d'impulsions d'un courant constant de quelques centaines de microampères à une fréquence de quelques hertz, entre deux électrodes disposées dans la cage thoracique, ou bien entre le boîtier du dispositif implanté et une électrode, par exemple une électrode de stimulation, comme enseigné par exemple par le US-A-4 567 892. Les variations d'impédance reproduisent les variations du volume thoracique, avec des pics d'impédance lors de l'inspiration, lorsque les poumons sont remplis d'air, et une impédance décroissante lors de la phase expiratoire.

Il a toutefois été constaté en pratique, lors d'études cliniques, qu'un système mettant en oeuvre un capteur d'activité respiratoire enregistrant les variations de volume pulmonaire au niveau thoracique peut être leurré dans certaines circonstances, conduisant à des risques de faux positif et de faux négatif susceptibles de perturber l'interprétation des signaux par le dispositif et de conduire ainsi à un diagnostic erroné.

Certaines de ces perturbations peuvent être éliminées par des filtrages appropriés, de manière en elle-même connue.

Ainsi, l'impédance transthoracique varie en fonction de la résistivité des tissus au moment de l'injection de l'impulsion de courant ; comme cette résistivité dépend essentiellement de la quantité d'air dans les poumons et de la quantité de sang dans les cavités cardiaques, le signal d'impédance recueilli est modulé à la fois par la respiration et par la fréquence cardiaque. L'impédance est également modulée par les variations de distance entre l'électrode de mesure et le boîtier du dispositif, distance qui varie cycliquement en fonction des battements cardiaques.

Ces composantes cardiaques peuvent être éliminées par un simple filtrage passe-bas, la fréquence cardiaque étant dans la plupart des cas de l'ordre de trois fois supérieure à la fréquence respiratoire.

D'autre part, afin de n'extraire du signal que la composante respiratoire (variation dynamique, seule significative), il convient d'éliminer l'impédance statique liée à l'impédance des tissus constatée dans une position corporelle stable et en l'absence de toute respiration et de tout battement cardiaque.

Cette composante statique est éliminée par mise en oeuvre d'un filtrage de type passe-haut.

Lorsque l'impédance transthoracique est utilisée pour estimer la ventilation minute et réaliser un asservissement de la fréquence cardiaque, cette estimation de la ventilation-minute repose sur un moyennage de plusieurs cycles respiratoires successifs, de sorte que l'impact de quelques cycles respiratoires artéfactés reste faible.

Le point de départ de l'invention réside dans la constatation de certains phénomènes pouvant perturber la mesure de l'impédance transthoracique, qui ne sont pas éliminés par les filtrages passe-bas et passe-haut existants.

En particulier, si l'on souhaite utiliser l'impédance transthoracique pour suivre les variations instantanées de l'activité respiratoire, notamment pour détecter des apnées et des hypopnées, il est indispensable d'éliminer tous les cycles artéfactés, au risque de produire des faux positifs et des faux négatifs conduisant à des diagnostics erronés de troubles respiratoires du patient.

Ainsi, l'impédance transthoracique peut être modifiée par un mouvement du patient, ou bien varier sous l'effet des contractions du diaphragme au cours d'une apnée obstructive. Ces phénomènes vont produire des artéfacts qui vont perturber le système et peuvent conduire à une détection erronée de cycles respiratoires particulièrement amples ou rapides, ou au contraire d'amplitude faible et/ou de périodes longues, pouvant conduire à un faux positif.

Un autre type d'artéfact peut résulter de la présence dans le signal d'impédance d'une composante liée aux battements cardiaques et non éliminée par les filtres passe-haut existants.

En effet, dans certaines circonstances (par exemple en situation à la fois de bradycardie et d'hyperventilation), la fréquence respiratoire et la fréquence cardiaque peuvent devenir suffisamment proches pour que les battements cardiaques influencent de façon significative le signal d'impédance. Le rythme cardiaque peut se trouver alors interprété à tort comme un rythme respiratoire, risquant ainsi de masquer la présence d'une apnée ou d'une hypopnée (faux négatif, au moment au moment où un véritable évènement pathologique respiratoire survient).

Dans le cas d'un appareil externe (polysomnographie), ces perturbations peuvent être détectées par la personne chargée d'interpréter les signaux, qui peut décider de ne pas prendre en compte une ou plusieurs des voies lorsque le signal apparaît bruité.

Mais cette appréciation subjective des signaux n'est pas envisageable dans un appareil implanté, qui fonctionne de façon entièrement automatique, selon des règles définies par un algorithme d'analyse.

L'un des buts de la présente invention est d'améliorer l'analyse de l'activité respiratoire par un dispositif implanté, notamment de manière à éliminer les faux positifs et les faux négatifs résultant de cycles artéfactés non pris en compte par les moyens de filtrage connus.

La détection des cycles artéfactés selon l'invention s'applique très avantageusement aux dispositifs implantés comprenant des moyens de diagnostic et de traitement des troubles respiratoires, qui nécessitent un suivi cycle à cycle de l'activité respiratoire du patient, de manière à pouvoir diagnostiquer sans délai la survenue d'un évènement respiratoire pathologique.

A cet effet, l'invention propose un dispositif médical comprenant de manière en elle-même connue (par exemple d'après le EP-A-0 970 713 précité) les éléments énoncés dans le préambule de la revendication 1. Selon l'invention, le dispositif comprend également les moyens énoncés dans la partie caractérisante de la revendication 1. Les sous-revendications visent des formes particulières de mise en oeuvre.

On va maintenant décrire de façon plus détaillée un exemple de mise en oeuvre de l'invention.

L'invention peut notamment être appliquée aux dispositifs commercialisé par ELA Médical, Montrouge, France. Il s'agit de dispositifs à microprocesseur comportant des circuits pour recevoir, mettre en forme et traiter des signaux électriques recueillis par des électrodes implantées. Ils est possible d'y charger par télémétrie des logiciels qui seront conservés en mémoire et exécutés pour mettre en oeuvre les fonctions de l'invention qui seront décrites ci-dessous.

L'invention est applicable à un dispositif implanté comportant des moyens pour détecter la survenue d'apnées ou d'hypopnées par analyse du rythme respiratoire du patient pendant son sommeil, ce rythme étant donné par l'évolution au cours du temps du signal de ventilation-minute (signal MV).

Le signal MV est un paramètre à prépondérance physiologique obtenu par une mesure transthoracique d'impédance. Cette mesure est opérée entre deux électrodes disposées dans la cage thoracique, ou entre une électrode (par exemple une électrode de stimulation, si le dispositif implanté est un stimulateur cardiaque) et le boîtier du dispositif. L'impédance est mesurée par injection d'un courant constant de quelques centaines de microampères, à une fréquence de quelques hertz, typiquement 8 Hz. Cette technique est par exemple décrite par Bonnet JL et coll., Measurement of Minute-Ventilation with Different DDDR Pacemaker Electrode Configurations, PACE, Vol. 21, 98, Part 1, et elle est mise en oeuvre dans les appareils de la série *Chorus RM 7034* d'ELA Médical.

Le dispositif considère qu'il y a apnée lorsqu'il détecte un arrêt respiratoire de durée supérieure à 10 secondes, phénomène qu'il est aisé de détecter par suivi du signal MV. Pour détecter les hypopnées, le dispositif peut par exemple comparer entre elles des moyennes glissantes du signal MV, établies par exemple sur une durée de 10 secondes : si entre deux moyennes consécutives une décroissance importante de la ventilation-minute est détectée, par exemple de plus de 50 %, alors le dispositif considère qu'il y a eu hypopnée.

Comme indiqué plus haut, l'invention propose de mettre en oeuvre une surveillance du signal respiratoire de manière à identifier certaines catégories d'artéfacts et engager des actions préventives lors du diagnostic.

La première série d'artéfacts prise en compte par l'invention sont ceux liés au mouvement, qui sont probablement les plus nombreux et conduisent généralement à une modification soudaine de l'impédance statique.

A cet effet, selon l'invention, le dispositif comporte dans la chaîne de mesure de l'impédance transthoracique des moyens pour identifier un saut d'impédance statique.

Pour cela, le logiciel du dispositif peut scruter en temps réel un ou plusieurs paramètres de la chaîne de mesure afin de détecter notamment :
- la saturation d'un amplificateur,
- des valeurs extrêmes atteintes dans la chaîne de numérisation, ou
- la modification d'une fréquence de coupure dans le filtrage du signal.

Si un saut d'impédance statique est détecté, le dispositif réagit en suspendant le diagnostic d'un trouble respiratoire du sommeil tant que le signal n'est pas stabilisé à sa nouvelle valeur, c'est-à-dire pendant le temps nécessaire au filtrage de la nouvelle composante statique.

Il est possible d'introduire une temporisation avant de réactiver la fonction de diagnostic.

Selon un autre aspect de l'invention, les artéfacts peuvent également être identifiés à partir de leurs conséquences sur la détection des cycles respiratoires, à partir de l'amplitude et/ou de la période de ces cycles, et/ou de la variation cycle à cycle de ces paramètres.

En effet, on constate en pratique que la période respiratoire est généralement assez stable, et présente donc des variations plutôt lentes. Ainsi, lorsqu'une apnée survient, une variation importante de la période respiratoire apparaît, mais cette période passe directement d'une valeur normale (typiquement 3 à 4 secondes) à une valeur élevée (supérieure à 10 secondes, conformément à la définition de l'apnée).

De ce fait, des variations de périodes de l'ordre de plusieurs secondes, mais inférieures à 10 secondes, peuvent laisser suspecter un artéfact.

On peut également définir la durée minimum d'un cycle respiratoire (par exemple 2 secondes), tout cycle de durée inférieure pouvant laisser suspecter un artéfact.

Il est également possible d'analyser l'amplitude des cycles respiratoires. La chaîne de mesure de la ventilation-minute est en effet dimensionnée pour pouvoir mesurer des cycles respiratoires amples, survenant au cours d'un effort par exemple. Mais lorsqu'un cycle ample survient de façon parfaitement isolée, il est là encore possible de suspecter un artéfact.

Pour tenir compte de ces situations, l'algorithme selon l'invention analyse les cycles respiratoires successifs après les avoir déterminés et classifiés (amplitude, période).

Tout cycle respiratoire répondant à l'un des critères suivants sera alors considéré comme artéfacté :
- la période du cycle est inférieure ou égale à *x* secondes (par exemple inférieure à *x* = 2 secondes),
- la période du cycle est inférieure à 10 secondes et présente un accroissement de *y* secondes (par exemple *y* = 4 secondes) par rapport à la période du cycle précédent,
- l'amplitude du cycle a augmenté de *z* unités par rapport au cycle précédent, dans le cas où ce cycle précédent ne coïncide pas avec un diagnostic d'apnée ou d'hypopnée (car dans ce dernier cas une reprise ventilatoire importante est attendue).

Le paramètre *z* peut prendre une valeur prédéterminée (par exemple *z* = 50 mV) ou bien une valeur relative, calculée sur un nombre représentatif de cycles respiratoires (par exemple un accroissement de *z* = 25% par rapport à la moyenne des amplitudes mesurées au cours de la journée précédente).

En présence de cycles considérés comme artéfactés, le dispositif peut réagir de diverses manières.

Tout d'abord, ce cycle artéfacté peut être simplement écarté de la gestion des diverses fonctions du dispositif (typiquement, de la fonction d'asservissement) et il ne sera pas pris en compte pour la mise à jour de la moyenne des cycles respiratoires représentative du rythme courant, moyenne qui sera utilisée pour détecter les hypopnées.

Une autre action consiste à inhiber les moyens de diagnostic des troubles respiratoires en cas de cycle(s) artéfacté(s), de manière à suspendre la recherche d'une apnée ou d'une hypopnée, de la même manière que sur détection d'un saut d'impédance statique.

Cette inhibition du diagnostic peut être déclenchée soit sur identification d'un cycle artéfacté isolé, soit seulement en cas de répétition trop fréquente des cycles artéfactés.

Par exemple, l'algorithme détermine à chaque cycle le nombre de cycles artéfactés survenus au cours des 16 derniers cycles respiratoires (fenêtre glissante) ; si ce compte atteint un seuil déterminé, alors l'algorithme invalide les apnées ou hypopnées éventuellement diagnostiquées au cours de ces cycles précédents.

En variante ou en complément, si le système dispose de plusieurs capteurs pour la détection des troubles respiratoires (par exemple un capteur de saturation en oxygène en plus de la chaîne de mesure d'impédance transthoracique), l'algorithme peut n'inhiber que la recherche des apnées ou hypopnées basée sur le capteur d'impédance transthoracique, en poursuivant la recherche des troubles respiratoires à partir du seul signal de saturation en oxygène.

## Revendications

1. Un dispositif médical implantable actif tel que stimulateur cardiaque, défibrillateur et/ou cardioverteur, ou dispositif multisite, du type comprenant:
- des moyens de mesure d'impédance transthoracique, délivrant un signal d'impédance variant avec l'activité respiratoire du patient,
- des moyens de traitement du signal d'impédance aptes à délivrer, à partir de ce signal, un signal respiratoire présentant une succession de cycles respiratoires consécutifs et représentatif de l'activité respiratoire du patient,
- des moyens de diagnostic de trouble respiratoire, aptes à analyser les variations du signal respiratoire sur une pluralité de cycles successifs, et
- des moyens de détection automatique de cycles respiratoires artéfactés, compartant des moyens aptes à identifier, dans un cycle respiratoire ou une séquence de cycles respiratoires, une singularité prédéterminée représentative d'un cycle artéfacté, dispositif **caractérisé en ce que** lesdits moyens de détection automatique des cycles respiratoires artéfactés comprennent en outre des moyens aptes à identifier, dans le signal d'impédance, un saut d'impédance statique.

2. Le dispositif de la revendication 1, dans lequel lesdits moyens aptes à identifier un saut d'impédance statique comprennent des moyens aptes à détecter la saturation d'un étage amplificateur du circuit des moyens de traitement du signal d'impédance.

3. Le dispositif de la revendication 1, dans lequel lesdits moyens aptes à identifier un saut d'impédance statique comprennent des moyens aptes à détecter le franchissement d'une valeur de seuil dans un étage de numérisation du circuit des moyens de traitement du signal d'impédance.

4. Le dispositif de la revendication 1, dans lequel lesdits moyens aptes à identifier un saut d'impédance statique comprennent des moyens aptes à détecter une modification de la fréquence de coupure desdits moyens de filtrage passe-haut et/ou passe-bas.

5. Le dispositif de la revendication 1, dans lequel lesdits moyens aptes à identifier une singularité prédéterminée comprennent des moyens aptes à détecter un cycle respiratoire dont la période est inférieure à une première limite.

6. Le dispositif de la revendication 5, dans lequel ladite première limite est de 2 secondes.

7. Le dispositif de la revendication 1, dans lequel lesdits moyens aptes à identifier une singularité prédéterminée comprennent des moyens aptes à détecter un cycle respiratoire dont la période est inférieure à une deuxième limite et présentant un accroissement supérieur à une troisième limite par rapport à la période du cycle précédent.

8. Le dispositif de la revendication 7, dans lequel ladite deuxième limite est de 10 secondes et ladite troisième limite est de 4 secondes.

9. Le dispositif de la revendication 1, dans lequel lesdits moyens aptes à identifier une singularité prédéterminée comprennent des moyens aptes à détecter un cycle respiratoire dont l'amplitude présente un accroissement supérieur à une quatrième limite par rapport à l'amplitude du cycle précédent, et pour lequel les moyens de diagnostic n'ont pas détecté de trouble respiratoire au cycle précédent.

10. Le dispositif de la revendication 9, dans lequel ladite quatrième limite est une valeur prédéterminée.

11. Le dispositif de la revendication 10, dans lequel ladite quatrième limite est de 50 millivolts.

12. Le dispositif de la revendication 9, dans lequel ladite quatrième limite est une valeur relative, déterminée par rapport à une moyenne des amplitudes mesurées sur une période antérieure.

13. Le dispositif de la revendication 12, dans lequel ladite quatrième limite est de +25 % par rapport à la moyenne des amplitudes mesurées au cours d'une journée précédente.

14. Le dispositif de la revendication 1, dans lequel les moyens de détection automatique de cycles respiratoires artéfactés sont en outre aptes à inhiber les moyens de diagnostic de trouble respiratoire en cas d'identification d'un saut d'impédance statique et/ou d'une singularité prédéterminée.

15. Le dispositif de la revendication 1, dans lequel les moyens de détection automatique de cycles respiratoires artéfactés sont en outre aptes à inhiber les moyens de diagnostic pendant une durée minimale de temporisation donnée après identification d'un saut d'impédance statique et/ou d'une singularité prédéterminée.

16. Le dispositif de la revendication 1, dans lequel les moyens de diagnostic sont également aptes à calculer et mettre à jour une moyenne des périodes des cycles respiratoires, et dans lequel les moyens de détection automatique de cycles respiratoires artéfactés sont en outre aptes à inhiber la mise à jour de ladite moyenne en cas d'identification d'un saut d'impédance statique et/ou d'une singularité prédéterminée.

17. Le dispositif de la revendication 1, dans lequel les moyens de détection automatique de cycles respiratoires artéfactés sont en outre aptes à invalider ledit profil de variation prédéterminé éventuellement détecté par les moyens de diagnostic en cas d'identification de sauts d'impédance statique et/ou de singularités prédéterminées dont le nombre dépasse une cinquième limite donnée au cours d'une séquence de cycles antérieurs.

18. Le dispositif de la revendication 17, dans lequel ladite cinquième limite donnée est de quatre pour une séquence de seize cycles antérieurs.

## Claims

1. An active implantable medical device such as a cardiac pacemaker, defibrillator and/or cardioverter, or multisite device, comprising:
- means for measuring a transthoracic impedance, for delivering an impedance signal varying with the respiratory activity of the patient;
- impedance signal processing means, for issuing from said signal a respiratory signal with a succession of consecutive respiratory cycles and being representative of the respiratory activity of the patient;
- respiratory disorder diagnosis means, for analyzing the variations of the respiratory signal over a plurality of successive cycles; and
- means for automatically detecting respiratory cycles with artifacts, comprising means for identifying a predetermined singularity representative of a cycle with artifact in at least one respiratory cycle or in a sequence of respiratory cycles,
said device being **characterized in that** said means for automatically detecting respiratory cycles with artifacts further comprises means for identifying a static impedance jump in the impedance signal.

2. The device of claim 1, wherein said means for identifying a static impedance jump comprises means for detecting the saturation of an amplifying stage of the impedance processing means circuit.

3. The device of claim 1, wherein said means for identifying a static impedance jump comprises means for detecting the crossing of a threshold value in a digitization stage of the impedance processing means circuit.

4. The device of claim 1, wherein said means for identifying a static impedance jump comprises means for detecting a modification of the cutoff frequency of high-pass and/or low-pass filtering means.

5. The device of claim 1, wherein said means for identifying a predetermined singularity comprises means for detecting a respiratory cycle having a period shorter than a first limit.

6. The device of claim 5, wherein said first limit is 2 seconds.

7. The device of claim 1, wherein said means for identifying a predetermined singularity comprises means for detecting a respiratory cycle having a period shorter than a second limit and with an increase higher than a third limit with respect to the period of the preceding cycle.

8. The device of claim 7, wherein said second limit is 10 seconds and said third limit is 4 seconds.

9. The device of claim 1, wherein said means for identifying a predetermined singularity comprises means for detecting a respiratory cycle having an amplitude with an increase higher than a fourth limit with respect to the amplitude of the preceding cycle, and for which cycle the diagnosis means has not detected a respiratory disorder in the preceding cycle.

10. The device of claim 9, wherein said fourth limit is a predetermined value.

11. The device of claim 10, wherein said fourth limit is 50 mV.

12. The device of claim 9, wherein said fourth limit is a relative value, determined with respect to an average of the amplitudes measured over an earlier period.

13. The device of claim 12, wherein said fourth limit is +25% wit respect to the average of the amplitudes measured over the previous day.

14. The device of claim 1, wherein said means for automatically detecting respiratory cycles with artifacts is further adapted to inhibit the respiratory disorder diagnosis means in the event of identification of a static impedance jump and/or a predetermined singularity.

15. The device of claim 1, wherein said means for automatically detecting respiratory cycles with artifacts is further adapted to inhibit the respiratory disorder diagnosis means during one minimal, given delay duration after the identification of a static impedance jump and/or of a predetermined singularity.

16. The device of claim 1, wherein said diagnosis means is further adapted to calculate and update an average of the periods of the respiratory cycles, and wherein the means for automatically detecting respiratory cycles with artifacts is further adapted to inhibit the update of said average in the event of identification of a static impedance jump and/or of a predetermined singularity.

17. The device of claim 1, wherein said means for automatically detecting respiratory cycles with artifacts is further adapted to invalidate the predetermined variation profile possibly detected by the diagnosis means in the event of identification of static impedance jumps and/or of predetermined singularities the number of which exceeds a fifth, given limit over a sequence of previous cycles.

18. The device of claim 17, wherein said fifth, given limit is four, for a sequence of sixteen previous cycles.

## Patentansprüche

1. Aktive implantierbare medizinische Vorrichtung, wie z.B. Herzschrittmacher, Defibrillator und/oder Kardioverter, oder Multisite-Vorrichtung, vom Typ mit:
- Mitteln zur Messung der transthorakalen Impedanz, die ein Impedanzsignal liefern, das mit der Atmungsaktivität des Patienten variiert,
- Mitteln zur Verarbeitung des Impedanzsignals, die geeignet sind, anhand dieses Signals ein Atmungssignal zu liefern, das eine Abfolge von aufeinander folgenden Atmungszyklen aufweist und für die Atmungsaktivität des Patienten repräsentativ ist,
- Mitteln zur Diagnose einer Atmungsstörung, die geeignet sind, die Veränderungen des Atmungssignals über eine Mehrzahl von aufeinander folgenden Zyklen zu analysieren, und
- Mitteln zur automatischen Erfassung von mit Artefakten versehenen Atmungszyklen, mit Mitteln, die geeignet sind, in einem Atmungszyklus oder einer Sequenz von Atmungszyklen eine vorbestimmte für einen mit Artefakten versehenen Zyklus repräsentative Singularität zu identifizieren,
Vorrichtung, die **dadurch gekennzeichnet ist, dass** die Mittel zur automatischen Erfassung der mit Artefakten versehenen Atmungszyklen außerdem Mittel umfassen, die geeignet sind, im Impedanzsignal einen Sprung der statischen Impedanz zu identifizieren.

2. Vorrichtung nach Anspruch 1, bei welcher die Mittel, die geeignet sind, einen Sprung der statischen Impedanz zu identifizieren, Mittel umfassen, die geeignet sind, die Sättigung einer Verstärkerstufe der Schaltung der Mittel zur Verarbeitung des Impedanzsignals zu erfassen.

3. Vorrichtung nach Anspruch 1, bei welcher die Mittel, die geeignet sind, einen Sprung der statischen Impedanz zu identifizieren, Mittel umfassen, die geeignet sind, das Durchqueren eines Schwellenwerts in einer Digitalisierungsstufe der Schaltung der Mittel zur Verarbeitung des Impedanzsignals zu erfassen.

4. Vorrichtung nach Anspruch 1, bei welcher die Mittel, die geeignet sind, einen Sprung der statischen Impedanz zu identifizieren, Mittel umfassen, die geeignet sind, eine Veränderung der Grenzfrequenz der Hochpass- und/oder Tiefpass-Filtermittel zu erfassen.

5. Vorrichtung nach Anspruch 1, bei welcher die Mittel, die geeignet sind, eine vorbestimmte Singularität zu identifizieren, Mittel umfassen, die geeignet sind, einen Atmungszyklus zu erfassen, dessen Periode geringer als eine erste Grenze ist.

6. Vorrichtung nach Anspruch 5, bei welcher die erste Grenze 2 Sekunden beträgt.

7. Vorrichtung nach Anspruch 1, bei welcher die Mittel, die geeignet sind, eine vorbestimmte Singularität zu identifizieren, Mittel umfassen, die geeignet sind, einen Atmungszyklus zu erfassen, dessen Periode geringer als eine zweite Grenze ist und eine Zunahme aufweist, die im Verhältnis zur Periode des vorangehenden Zyklus größer als eine dritte Grenze ist.

8. Vorrichtung nach Anspruch 7, bei welcher die zweite Grenze 10 Sekunden beträgt und die dritte Grenze 4 Sekunden beträgt.

9. Vorrichtung nach Anspruch 1, bei welcher die Mittel, die geeignet sind, eine vorbestimmte Singularität zu identifizieren, Mittel umfassen, die geeignet sind, einen Atmungszyklus zu erfassen, dessen Amplitude eine Zunahme aufweist, die im Verhältnis zur Amplitude des vorangehenden Zyklus größer als eine vierte Grenze ist, und bei welcher die Diagnosemittel beim vorherigen Zyklus keine Atmungsstörung erfasst haben.

10. Vorrichtung nach Anspruch 9, bei welcher die vierte Grenze ein vorbestimmter Wert ist.

11. Vorrichtung nach Anspruch 10, bei welcher die vierte Grenze 50 Millivolt beträgt.

12. Vorrichtung nach Anspruch 9, bei welcher die vierte Grenze ein relativer Wert ist, der im Verhältnis zu einem Durchschnitt der über eine frühere Periode gemessenen Amplituden bestimmt wird.

13. Vorrichtung nach Anspruch 12, bei welcher die vierte Grenze über 25% im Verhältnis zum Durchschnitt der im Verlauf eines vorherigen Tages gemessenen Amplituden liegt.

14. Vorrichtung nach Anspruch 1, bei welcher die Mittel zur automatischen Erfassung von mit Artefakten versehenen Atmungszyklen außerdem geeignet sind, die Mittel zur Diagnose einer Atmungsstörung im Falle der Identifizierung eines Sprungs der statischen Impedanz und/oder einer vorbestimmten Singularität zu unterdrücken.

15. Vorrichtung nach Anspruch 1, bei welcher die Mittel zur automatischen Erfassung von mit Artefakten versehenen Atmungszyklen außerdem geeignet sind, die Diagnosemittel während einer minimalen gegebenen Verzögerungsdauer nach Identifizierung eines Sprungs der statischen Impedanz und/oder einer vorbestimmten Singularität zu unterdrücken.

16. Vorrichtung nach Anspruch 1, bei welcher die Diagnosemittel ebenfalls geeignet sind, einen Durchschnitt der Perioden der Atmungszyklen zu berechnen und zu aktualisieren, und bei welcher die Mittel zur automatischen Erfassung von mit Artefakten versehenen Atmungszyklen außerdem geeignet sind, die Aktualisierung des Durchschnitts Im Falle der Identifizierung eines Sprungs der statischen Impedanz und/oder einer vorbestimmten Singularität zu unterdrücken.

17. Vorrichtung nach Anspruch 1, bei welcher die Mittel zur automatischen Erfassung von mit Artefakten versehenen Atmungszyklen außerdem geeignet sind, das vorbestimmte Änderungsprofil, das eventuell durch die Diagnosemittel erfasst wurde, im Falle der Identifizierung von Sprüngen der statischen Impedanz und/oder vorbestimmter Singularitäten, deren Anzahl eine fünfte gegebene Grenze im Verlauf einer Sequenz an vorherigen Zyklen übersteigt, zu verwerfen.

18. Vorrichtung nach Anspruch 17, bei welcher die fünfte gegebene Grenze bei einer Sequenz von sechzehn vorherigen Zyklen vier beträgt.
